# EUROPEAN PATENT APPLICATION

(11) **EP 2 952 175 A1**
(43) Date of publication of application: **09.12.2015**
(21) Application number: 15170443.4
(22) Date of filing: 03.06.2015
(51) Int. Cl.: A61K 9/00, A61K 9/20, A61K 31/00

(54) **A PROCESS FOR PREPARING A FLOTATION TABLET WITH PROLONGED ACTIVITY OF THERAPEUTIC SUBSTANCES ACTING ON A MUCOUS MEMBRANE OF THE STOMACH AND PHARMACEUTICAL COMPOSITION**

(30) Priority: 03.06.2014 PL 40842014
(71) Applicant: Uniwersytet Medyczny Im. Piastów Slaskich We Wroclawiu, 50-367 Wroclaw (PL)
(72) Inventor: Musia , Witold, 50-150 Wroc aw (PL); Dry , Andrzej, 50-349 Wroc aw (PL); Golonka, Iwona, 55-231 Wroc aw (PL); Wójcik-Pastuszka, Dorota, 55-011 Siechnice (PL)
(74) Representative: Witek, Rafal

(57) **Abstract**

The object of the present invention is a method of manufacturing a floating tablet with extended interaction of therapeutic substances with stomach mucosa, comprising the steps of weighing therapeutic substances, sugar alcohols, acid polymers and anti-adhesion and lubricating substances, mixing them together, pouring into the tablet press hopper and pressing tablets having the diameter of 4 to 28 mm, using pressing force of 50 to 100,000, the therapeutic substance being preferably a compound selected from the group containing: pioglitazone hydrochloride, cephalexin, potassium losartan, captopril, diltiazem, chlorhexidine hydrochloride, and the acid polymer constitutes 0.5% - 94.9%wt. of the mixture, sugar alcohols constitute 5.0 - 99.4%wt. of the mixture, anti-adhesive and lubricating substances constitute 0.1% - 0.5%wt. of the mixture, The object of the present invention is also a pharmaceutic composition containing a therapeutic substance, sugar alcohols, acidic polymers of organic acids, and anti-adhesive and lubricating substances, the therapeutic substance being preferably a compound selected from the group containing: pioglitazone hydrochloride, cephalexin, potassium losartan, captopril, diltiazem, and the acid polymer constitutes 0.5% - 94.9%wt. of the composition, sugar alcohols constitute 5.0 - 99.4%wt. of the composition, anti-adhesive and lubricating substances constitute 0.1%-1.5%wt. of the composition,

## Description

The present invention relates to a method of manufacturing a floating tablet with extended interaction of therapeutic substances with stomach mucosa, and pharmaceutic composition for such tablet, finding application particularly in treatment of stomach conditions.

Currently, floating tablets are manufactured in a way that allows obtaining preparation of the density lower than the density of water, which makes such tablets float on the surface of the stomach contents or close to that surface. For that purpose, a mixture of appropriate polymers is used, such as cellulose derivatives, with the active substance whose apparent density is lower than that of water. Sodium or potassium bicarbonate is also added to the mixture, and often an organic acid as well. Carbon dioxide produced on contact with water provides floating of such tablet in the stomach environment. Floating tablets are with 30 mg pioglitazone hydrochloride are known, produced by means of wet powders granulation using isopropyl alcohol. Next, the dried granulate undergoes tableting. After placing the tablets in the stomach liquid environment, they undergo flotation by means of sodium bicarbonate reaction with the hydrochloric acid present in the stomach liquid, which provides prolonged presence of pioglitazone in the stomach environment, and may influence favorable extension of the medicine's interaction with the stomach mucosa. Known floating tablets are tablets with captopril 25 mg, which are manufactured by dry direct compression of two powder mixtures, one of which including e.g. sodium bicarbonate and citric acid, and the other contains, among others, the therapeutic substance. After placing the tablets in the stomach liquid environment, they undergo flotation by means of sodium bicarbonate reaction with the citric acid in the aqueous environment of the stomach liquid, which provides prolonged presence of captopril in the stomach environment, and may influence favorable extension of the medicine's interaction with the stomach mucosa. Known floating tablets are tablets with cefalexin 500 mg, produced by means of wet powder mixture granulation using isopropyl alcohol. Next, the dried granulate undergoes tableting. After placing the tablets in the stomach liquid environment, they undergo flotation by means of sodium bicarbonate reaction with the citric acid in the aqueous environment of the stomach liquid, which provides prolonged presence of cefalexin in the stomach environment, and may influence favorable extension of the medicine's interaction with the stomach mucosa. Known floating tablets are tablets with stavudine 80 mg, which are manufactured by dry direct compression of powder mixture, containing, among others, sodium bicarbonate and citric acid. After placing the tablets in the stomach liquid environment, they undergo flotation by means of sodium bicarbonate reaction with the citric acid in the aqueous environment of the stomach liquid, which provides prolonged presence of cefalexin in the stomach environment, and may influence favorable extension of the medicine's interaction with the stomach mucosa. Know floating tablets are tablets with losartan potassium salt 50 mg, produced by means of wet powder mixture granulation using isopropyl alcohol. Next, the dried granulate undergoes tableting. After placing the tablets in the stomach liquid environment, they undergo flotation by means of sodium bicarbonate reaction with the citric acid in the aqueous environment of the stomach liquid, which provides prolonged presence of losartan in the stomach environment, and may influence favorable extension of the medicine's interaction with the stomach mucosa.

All preparations referred to above are obtained with the application of a substance generating carbon dioxide, i.e. sodium bicarbonate, in acidic aqueous environment, originating from the organic acid introduced into the powder mixture, e.g. citric acid. After administering the drug to the patient, due to the reaction of replacing the weaker carbon acid by the stronger organic acid or hydrochloric acid, bubbles of carbon dioxide are formed, which may create a feeling of discomfort by the patient. Furthermore, in case of wet powder granulation, a significant obstacle in the production process is providing isolation of those powders from humidity in order to prevent generation of carbon dioxide already during the wet granulation process. Isopropyl alcohol used for that purpose is undesirable in terms of natural environment protection. Also storage of preparations containing components that react easily producing carbon dioxide is difficult and costly. Improper handling can easily lead to decomposition of active substances contained in such floating tablets. In case of using the system that does not comprise an organic acid, there is a hazard that, with patients with hypoacidity, the amount of generated carbon dioxide will be insufficient to provide floatation. In case of omitting the mixture of bicarbonate and organic acid form the composition of the tablet, the floatation and the mechanical properties of the tablet are insufficient to create an applicable system.

Polish patent No. PL149493 discloses a method to produce a tablet capable of floating on the surface of the gastric juice and releasing therapeutic factor in a prolonged period of time. The method referred to above consists in preparation of a solution of 0.5%-2% of gel forming agent in 33%-67% of water and cooling it down afterwards, and in preparation of a mixture containing 25%-50% of therapeutic substance, such as theophylline, ampicillin, or captopril, and 8%-15% of neutral, therapeutically acceptable oil. The obtained mixture is added to the said solution, poured into the tableting form, and left to rest until gel is formed, and then dried. The presented method is based on gel formation and drying of the floatation tablet components, which does not guarantee obtaining a mechanically stable product.

US patent application No. US5651985 discloses a composition containing a pharmacologically active compound dispersed on molecular level in a homogenous mixture of polyvinylpyrrolidone and methacrylic acid polymer, and, optionally a gas-forming additive. The application of the gas-forming additive enabled reducing the apparent density of the tablet, thus making it float on the surface of the gastric juice. The tablets did not fall apart in the aqueous medium and demonstrated swelling and stability in the swelling state. On the other hand, relatively low amounts of the gas forming agent were used in the presented method, and therefore there is a risk that the tablet will not float on the surface of the gastric juice in patients suffering from hypoacidity.

Further on, American patent application US5783212, discloses a pharmaceutic tablet of controlled release, comprising a first barrier layer comprising a swellable, erodible polymer, a drug layer comprising a second swellable, erodible polymer, and another barrier layer comprising another swellable, erodible polymer, wherein the said first and third barrier layers swell and erode faster than the drug layer. Such configuration increases drug delivery ratio from the onset of dissolution. A tablet was also disclosed, comprising a gas-forming agent in one of the layers, creating the floatation effect and extending the tablet retention time in the stomach. Such structure of the tablet requires multistage production process and may turn out to be ineffective with patients suffering from hypoacidity of gastric juices.

The technical problem faced by the present invention is to provide such production method of a floating tablet with limited absorption in alkaline environment of duodenum and small intestine in order to obtain extended interaction of therapeutic substances with the stomach mucosa, which will not require using an organic solvent unfavorable to natural environment, and the obtained product in the form of a tablet will have determined floatation and mechanical properties and higher durability of the therapeutic substance contained in the tablet and the tablet itself, and will provide the floatation effect even in patients with hypoacidity of gastric juice. Unexpectedly, the technical problems mentioned above have been solved by the present invention.

The first object of the present invention is a method of manufacturing a floating tablet with extended interaction of therapeutic substances with stomach mucosa, comprising the following steps:
a) weighing therapeutic substances, sugar alcohols, acid polymers and antiadhesive and lubricating substances in appropriate proportions, respectively to the desired number of tablets,
b) substances referred to in step a) are mixed together,
c) the mixture obtained in step b) is introduced into the tablet press hopper,
d) tablets are pressed,
the therapeutic substance being preferably selected from the group containing: pioglitazone hydrochloride, cephalexin, potassium losartan, captopril, diltiazem, chlorhexidine hydrochloride, characterized in that the acid polymer constitutes 0.5% - 94.9%wt. of the mixture, sugar alcohols constitute 5.0 - 99.4%wt. of the mixture, anti-adhesive and lubricating substances constitute 0.1% - 1.5%wt. of the mixture, and in step d) tablets with the diameter of 4 - 28 mm are pressed with the pressing force of 50 to 100,000 N. Preferably, the acid polymer has been selected from the group comprising synthetic polymers containing carboxyl group, including: polyacrylic acid, polymethacrylic acid, natural polymers containing carboxyl group, including alginic acid, pectic acid, hyaluronic acid, polymers containing sulfate groups, natural and synthetic, including chitin sulfate, chondroitin sulfate. Equally preferably sugar alcohols have been selected from a group comprising: glycerol, erythritol, xylitol, mannitol, sorbitol, perseitol, volemitol, β-sedoheptiol. In the preferred embodiment of the present invention, the anti-adhesive and lubricating substances have been selected from the group comprising: aluminum stearate, stearic acid, talc, polyoxyethylene glycols, magnesium or calcium stearate, silica. In another preferred embodiment of the present invention, step b) is carried out within 10 to 40 minutes.

The second object of the present invention is a pharmaceutic composition containing a therapeutic substance, sugar alcohols, acidic polymers of organic acids, and anti-adhesive and lubricating substances, the therapeutic substance being preferably a compound selected from the group containing: pioglitazone hydrochloride, cephalexin, potassium losartan, captopril, diltiazem, chlorhexidine hydrochloride, characterized in that the acid polymer constitutes 0.5% - 94.9%wt. of the composition, sugar alcohols constitute 5.0 - 99.4%wt. of the composition, anti-adhesive and lubricating substances constitute 0.1% - 1.5%wt. of the composition, Preferably, the acid polymer has been selected from the group comprising synthetic polymers containing carboxyl group, including: polyacrylic acid, polymethacrylic acid, natural polymers containing carboxyl group, including alginic acid, pectic acid, hyaluronic acid, polymers containing sulfate groups, natural and synthetic, including chitin sulfate, chondroitin sulfate. Equally preferably sugar alcohols have been selected from a group comprising: glycerol, erythritol, xylitol, mannitol, sorbitol, perseitol, volemitol, β-sedoheptiol. In the preferred embodiment of the present invention, the anti-adhesive and friction substances have been selected from the group comprising: aluminum stearate, stearic acid, talc, polyoxyethylene glycols, magnesium or calcium stearate, silica. In another preferred embodiment of the present invention, the composition has the form of a tablet.

The application of the method of floating tablet manufacturing according to the present invention will allow obtaining tablets of predefined floatation and mechanical properties without the necessity to implement mixtures of organic acids and bicarbonates sensitive to water. It provides a higher durability of the therapeutic substance contained in the tablet, as well as higher durability of the tablet itself. Furthermore, the method according to the present invention does not require wet granulation, which relates to the elimination of the organic solvent from the process as harmful to the natural environment. The method of obtaining floating tablets also provides reaching the floatation effect of the manufactured tablets, in patients with hypoacidity of gastric juice.

Exemplary embodiments of the invention have been presented in the drawings, wherein Fig. 1 illustrates a floating tablet floating at the surface of a liquid, fig. 2 illustrates another floating tablet floating on the surface of a liquid, fig. 3 illustrates a SEM image representing the microstructure of the floating tablet, and fig. 4 presents a diagram illustrating the comparison of the therapeutic substance quantity released in time by tablets according to the present invention.

### Example 1

The tablets were made of the following ingredients (ingredient weights in one tablet):

| | |
|---|---|
| Chlorhexidine hydrochloride, | 2.5 mg |
| Polyacrylic acid | 100 mg |
| Sorbitol | 500 mg |
| Magnesium stearate | 5 mg |

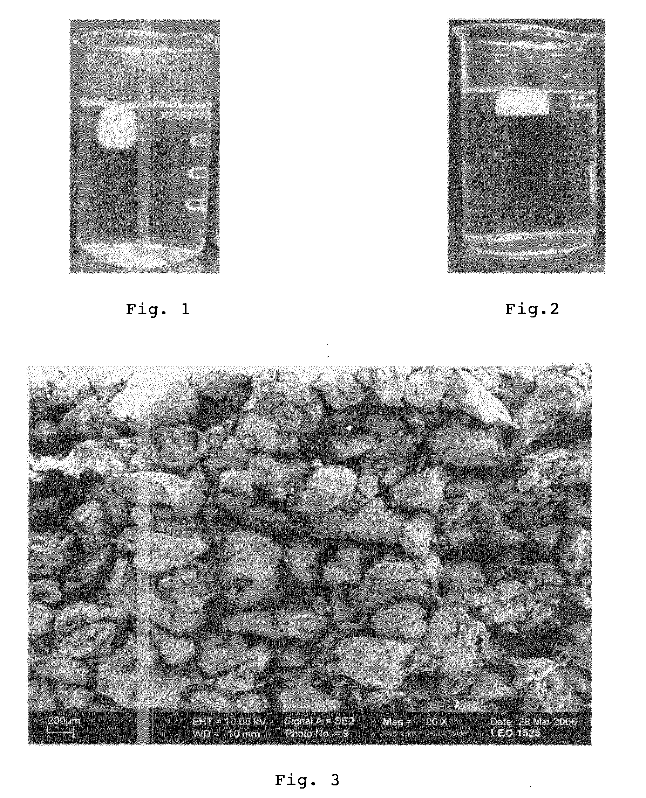

The ingredients, weighed proportionally for 10,000 tablets, were mixed in a dual cone mixer for 20 mins and then fed into the hopper of a rotary tablet press, and tablets with 8 mm diameter were pressed applying the force of 500 N. The floating tests of the tablet on the surface of gastric juice were carried out, and measurement with a scanning electron microscope taken. After placing the tablet in the gastric juice, sorbitol present in the cavities in the polymer framework is washed out, thus rapidly reducing the apparent density of the tablet below the density of the gastric juice, resulting in the tablet's floating on the surface where it releases the therapeutic substance. The image from the SEM is presented in Fig. 3 and illustrates the polymer framework of the floating tablet and sorbitol grains trapped in that structure.

### Example 2

The tablets were made of the following ingredients (ingredient weights in one tablet):

| | |
|---|---|
| Cephalexin | 500 mg |
| Polymethacrylic acid | 900 mg |
| Erythritol | 200 mg |
| Magnesium stearate | 10 mg |

The ingredients, weighed proportionally for 200,000 tablets, were mixed in a dual cone mixer for 40 mins and then fed into the hopper of a rotary tablet press, and tablets with 28 mm diameter were pressed with the force of 100000 N. The floating tests of the tablet on the surface of gastric juice were carried out. After placing the tablet in the gastric juice, erythritol present in the cavities in the polymer framework is washed out, thus rapidly reducing the apparent density of the tablet below the density of the gastric juice, resulting in the tablet's floating on the surface where it releases the therapeutic substance, which is presented in Fig. 2.

### Example 3

The tablets were made of the following ingredients (ingredient weights in one tablet):

| | |
|---|---|
| Potassium losartan | 50 mg |
| Alginic acid | 720 mg |
| Xylitol | 48 mg |
| Aluminum stearate | 7.5 mg |

The ingredients, weighed proportionally for 200 tablets, were mixed in a dual cone mixer for 10 mins and then fed into the hopper of a rotary tablet press, and tablets with 14 mm diameter were pressed with the force of 50000 N. The floating tests of the tablet on the surface of gastric juice were carried out. After placing the tablet in the gastric juice, xylitol present in the cavities in the polymer framework is washed out, thus rapidly reducing the apparent density of the tablet below the density of the gastric juice, resulting in the tablet's floating on the surface where it releases the therapeutic substance, which is presented in Fig. 1.

### Example 4

The tablets were made of the following ingredients (ingredient weights in one tablet):

| | |
|---|---|
| Captopril | 50 mg |
| Pectic acid | 25 mg |
| Mannitol | 480 mg |
| Stearic acid | 2.5 mg |

The ingredients, weighed proportionally for 50,000 tablets, were mixed in a dual cone mixer for 30 mins and then fed into the hopper of a rotary tablet press, and tablets with 6 mm diameter were pressed with the force of 500 N. The floating tests of the tablet on the surface of gastric juice were carried out. After placing the tablet in the gastric juice, mannitol present in the cavities in the polymer framework is washed out, thus rapidly reducing the apparent density of the tablet below the density of the gastric juice, resulting in the tablet's floating on the surface where it releases the therapeutic substance.

### Example 5

The tablets were made of the following ingredients (ingredient weights in one tablet):

| | |
|---|---|
| Diltiazem | 60 mg |
| Chondroitin sulfate | 45 mg |
| Perseitol | 380 mg |
| Talc | 1.5 mg |

The ingredients, weighed proportionally for 100 tablets, were mixed in a dual cone mixer for 10 mins and then fed into the hopper of a rotary tablet press, and tablets with 4 mm diameter were pressed with the force of 50 N. The floating tests of the tablet on the surface of gastric juice were carried out. After placing the tablet in the gastric juice, perseitol present in the cavities in the polymer framework is washed out, thus rapidly reducing the apparent density of the tablet below the density of the gastric juice, resulting in the tablet's floating on the surface

### Example 6

The tablets were made of the following ingredients (ingredient weights in one tablet):

| | |
|---|---|
| Chlorhexidine hydrochloride | 30 mg |
| Chitin sulfate | 80 mg |
| Volemitol | 250 mg |
| Calcium stearate | 1.5 mg |

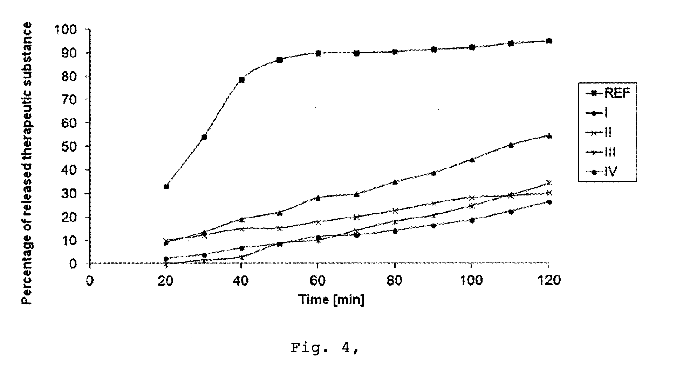

The tablets were made exactly like those of examples 1 - 5. The references REF, I, II, III, IV represent tablets with the model therapeutic substance - chlorhexidine hydrochloride, made with the application of various force in the tablet press. The quantity or the released therapeutic substance was evaluated with a validated spectrographic method. The release examination was carried out according to European Pharmacopeia, in a paddle apparatus for release testing. Fig. 4 illustrates the percentage of released model alkaline therapeutic substance of low solubility - chlorhexidine hydrochloride - from unmodified, classic, and typical tablets (REF) and modified tablets, according to the patent description (I, II, III, IV). As is clearly visible in the chart, the therapeutic substance from classic tablets (REF) is almost completely released after 50 min, while that of floating tablets, within the next hour, that is from 26.5% to 54,67% of the model therapeutic substance.

## Claims

1. Method of manufacturing a floating tablet with extended interaction of therapeutic substances with stomach mucosa, comprising the following steps:
a) weighing therapeutic substances, sugar alcohols, acid polymers and antiadhesive and friction substances in appropriate proportions, respectively to the desired number of tablets,
b) substances referred to in step a) are mixed together,
c) the mixture obtained in step b) is introduced into the tablet press hopper,
d) tablets are pressed,
the therapeutic substance being preferably selected from the group containing: pioglitazone hydrochloride, cephalexin, potassium losartan, captopril, diltiazem, chlorhexidine hydrochloride, **characterized in that** the acid polymer constitutes 0.5% - 94.9%wt. of the mixture, sugar alcohols constitute 5.0 - 99.4%wt. of the mixture, anti-adhesive and lubricating substances constitute 0.1% - 1.5%wt. of the mixture, and in step d) tablets with diameters from 4 - 28 mm are pressed with the pressing force from 50 to 1,000,000 N.

2. The method of claim 1, **characterized in that** the acid polymer has been selected from the group comprising synthetic polymers containing carboxyl group, including: polyacrylic acid, polymethacrylic acid, natural polymers containing carboxyl group, including alginic acid, pectic acid, hyaluronic acid, polymers containing sulfate groups, natural and synthetic, including chitin sulfate, chondroitin sulfate.

3. The method of claim 1 or 2, **characterized in that** sugar alcohols have been selected from a group comprising: glycerol, erythritol, xylitol, mannitol, sorbitol, perseitol, volemitol, and β-sedoheptiol.

4. The method of claims from 1 through 3, **characterized in that** the anti-adhesive and lubricating substances have been selected from the group comprising: aluminum stearate, stearic acid, talc, polyoxyethylene glycols, magnesium or calcium stearate, silica.

5. The method of claims from 1 through 4, **characterized in that** step b) is carried out for 10 to 40 minutes.

6. Pharmaceutic composition containing a therapeutic substance, sugar alcohols, acidic polymers of organic acids, and anti-adhesive and lubricating substances, the therapeutic substance being preferably a compound selected from the group containing: pioglitazone hydrochloride, cephalexin, potassium losartan, captopril, diltiazem, chlorhexidine hydrochloride, **characterized in that** the acid polymer constitutes 0.5% - 94.9%wt. of the composition, sugar alcohols constitute 5.0 - 99.4%wt. of the composition, anti-adhesive and lubricating substances constitute 0.1% - 1.5%wt. of the composition,

7. The composition of claim 6, **characterized in that** the acid polymer has been selected from the group comprising synthetic polymers containing carboxyl group, including: polyacrylic acid, polymethacrylic acid, natural polymers containing carboxyl group, including alginic acid, pectic acid, hyaluronic acid, polymers containing sulfate groups, natural and synthetic, including chitin sulfate, chondroitin sulfate.

8. The composition of claim 6 or 7, **characterized in that** sugar alcohols have been selected from a group comprising: glycerol, erythritol, xylitol, mannitol, sorbitol, perseitol, volemitol, and β-sedoheptiol.

9. The composition of claim from 6 through 8, **characterized in that** the anti-adhesive and lubricating substances have been selected from the group comprising: aluminum stearate, stearic acid, talc, polyoxyethylene glycols, magnesium or calcium stearate, silica.

10. The composition of claim from 6 through 9, **characterized in that** it has the form of a tablet.
